(19) Europäisches Patentamt
European Patent Office
Office européen des brevets

(11) **EP 4 365 622 A1**

(12) **EUROPEAN PATENT APPLICATION**
published in accordance with Art. 153(4) EPC

(43) Date of publication:
**08.05.2024 Bulletin 2024/19**

(21) Application number: **22833092.4**

(22) Date of filing: **27.06.2022**

(51) International Patent Classification (IPC):
*G01S 7/02* (2006.01)      *G01S 13/50* (2006.01)
*A61B 5/107* (2006.01)      *A61B 5/11* (2006.01)
*A61B 5/117* (2016.01)

(52) Cooperative Patent Classification (CPC):
**A61B 5/107; A61B 5/11; A61B 5/117; G01S 7/02;
G01S 13/50**

(86) International application number:
**PCT/JP2022/025557**

(87) International publication number:
**WO 2023/276945 (05.01.2023 Gazette 2023/01)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.06.2021 JP 2021108195**

(71) Applicant: **Panasonic Intellectual Property
Management Co., Ltd.
Kadoma-shi, Osaka 571-0057 (JP)**

(72) Inventors:
• **IIZUKA, Shoichi
Kadoma-shi, Osaka 571-0057 (JP)**
• **NAKAYAMA, Takeshi
Kadoma-shi, Osaka 571-0057 (JP)**
• **HONMA, Naoki
Morioka, Iwate 020-8550 (JP)**
• **HAYASHI, Teppei
Morioka, Iwate 020-8550 (JP)**
• **SHIRAKI, Nobuyuki
Morioka, Iwate 020-8550 (JP)**

(74) Representative: **Novagraaf International SA
Chemin de l'Echo 3
1213 Onex, Geneva (CH)**

(54) **ESTIMATION DEVICE AND ESTIMATION METHOD**

(57)      An estimation device (10A) includes a transmitter-receiver (e.g., 30A) that includes transmitting antenna elements and receivers arranged around a predetermined range that includes a first living body, memory (41) that stores a teaching signal that corresponds to M × N second reception signals obtained by causing each receiver to receive a second reception signal in advance, the second reception signal including a reflected signal obtained by a second transmission signal reflecting off a second living body, the second transmission signal being transmitted from the transmitting antennal element to the second living body, and a circuit (40) that includes a first vector calculator and an estimator, the first vector calculator calculating a teaching first vector from the teaching signal and a test first vector from M × N first reception signals obtained by causing each receivers to receive a first reception signal, the estimator calculating a plurality of features from the teaching first vector and the test first vector and estimating identification information on the first living body by a predetermined method using the calculated features.

FIG. 1

**Description**

[Technical Field]

The present disclosure relates to an estimation device and an estimation method.

[Background Art]

Technologies are known in which radio signals are transmitted to a living body and reflected signals of the radio signals are received to identify the living body or to estimate the orientation of the living body (e.g., Patent Literatures (PTLs) 1 and 2).

PTL 1 discloses a device for estimating an individual driver of an automobile by emitting electromagnetic waves to the driver and using reflected waves of the electromagnetic waves to extract heart beat signals and heart sound signals.

PTL 2 discloses a method of measuring the heart rate of a driver of an automobile, which serves as a subject, by using a plurality of transmitter-receivers on the subject.

PTL 3 discloses a 360-degree radiation pattern measuring device using a plurality of antennas on a subject.

PTL 4 discloses an identification device for identifying an individual subject by using a plurality of antennas provided in the vicinity of the subject.

PTL 5 discloses an estimation device for estimating the orientation of a living body by using a plurality of antennas provided in the vicinity of a subject.

PTL 6 discloses a technology for estimating the direction of a living body by emitting radio signals to the living body and receiving reflected signals of the radio signal.

[Citation List]

[Patent Literature]

[PTL 1]
Japanese Unexamined Patent Application Publication No. 2015-042293
[PTL 2]
Japanese Unexamined Patent Application Publication No. 2009-055997
[PTL 3]
Japanese Unexamined Patent Application Publication No. 2007-325621
[PTL 4]
Japanese Unexamined Patent Application Publication No. 2019-93104
[PTL 5]
Japanese Unexamined Patent Application Publication No. 2019-211458
[PTL 6]
Japanese Patent No. 6504546

[Summary of Invention]

[Technical Problem]

As disclosed in PTL 1, 2, 4, or 5, the identification of a living body or the estimation of the orientation of a living body using electromagnetic waves is in many cases conducted while antennas and a subject to be measured are located at relatively close distances from each other.

The constraint of such a short distance between the antennas and the subject to be measured raises no problems in the case of identifying an individual in a confined region such as a driver seat or a private room, but makes the technology difficult to use in scenes such as daily life.

The method disclosed in PTL 6 is able to estimate the direction or position of a person present in a region other than confined regions, but unable to identify a living body.

The present disclosure has been made in light of the circumstances described above, and it is an object of the present disclosure to provide an estimation device and an estimation method capable of identifying a living body by using electromagnetic waves even if a subject and antennas are distanced from each other.

[Solution to Problem]

To achieve the object described above, an estimation device according to one aspect of the present disclosure includes M transmitting antenna elements arranged around a predetermined range that includes a first living body, where M is an integer greater than or equal to 1, the M transmitting antenna elements each transmitting a first transmission signal to the predetermined range, N receivers arranged around the predetermined range and each including a receiving antenna element, where N is an integer greater than or equal to 3, the N receivers each receiving a first reception signal for a predetermined period of time by using the receiving antenna element, the first reception signal including a reflected signal obtained by the first transmission signal reflecting off the first living body, memory that stores a teaching signal that corresponds to M × N second reception signals obtained by causing each of the N receivers to receive a second reception signal in advance, the second reception signal including a reflected signal obtained by a second transmission signal reflecting off a second living body, the second transmission signal being transmitted from the M transmitting antenna elements to the second living body, a first vector calculator that calculates a teaching first vector from the teaching signal and a test first vector from M × N first reception signals obtained by causing each of the N receivers to receive the first reception signal, and an estimator that calculates a plurality of features from the teaching first vector and the test first vector and estimates identification information on the first living body by a predetermined method using the plurality of features calculated.

It is to be noted that general or specific aspects of the present disclosure may be implemented as systems, methods, integrated circuits, computer programs, or computer-readable recording media such as CD-ROMs, or may be implemented as any arbitrary combination of systems, methods, integrated circuits, computer programs, and recording mediums.

[Advantageous Effects of Invention]

The estimation device according to the present disclosure is capable of identifying a living body by using electromagnetic waves even if a subject and an antenna is distanced from each other.

[Brief Description of Drawings]

[FIG. 1]

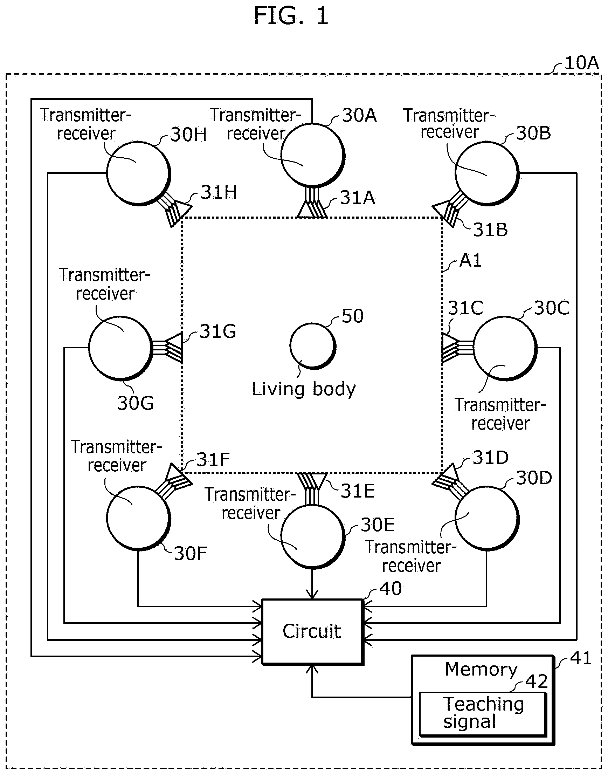

FIG. 1 is a schematic diagram showing one example of a configuration of an estimation device according to Embodiment 1.
[FIG. 2]

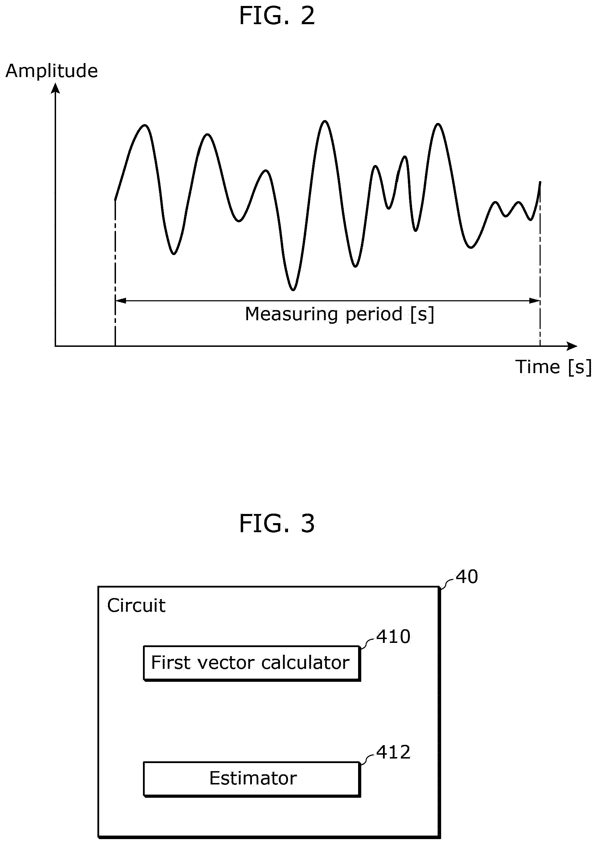

FIG. 2 is a diagram showing one example of a teaching signal shown in FIG. 1.
[FIG. 3]
FIG. 3 is a schematic diagram showing one example of a detailed configuration of a circuit according to Embodiment 1.
[FIG. 4]

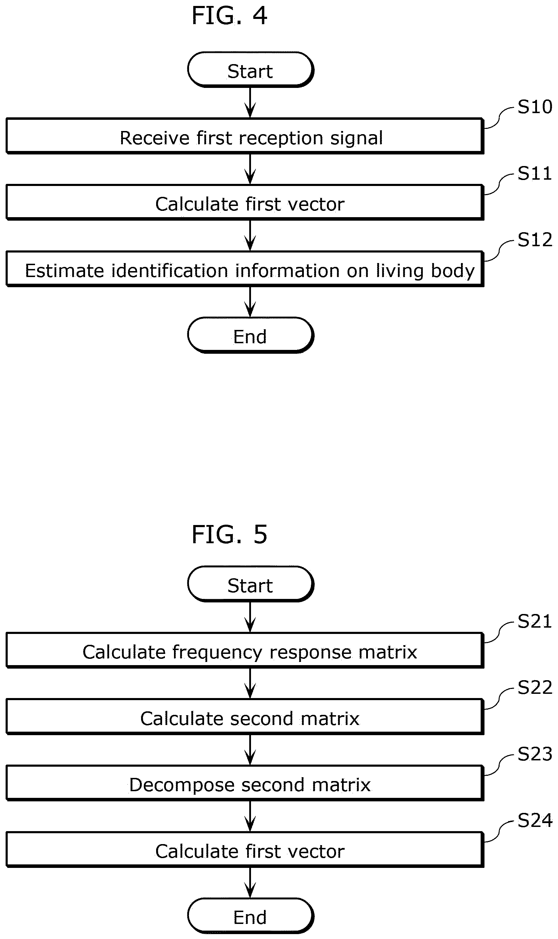

FIG. 4 is a flowchart showing one example of operations of the estimation device according to Embodiment 1.
[FIG. 5]
FIG. 5 is a flowchart showing a first example of the detailed operations of the estimation device according to Embodiment 1.
[FIG. 6]

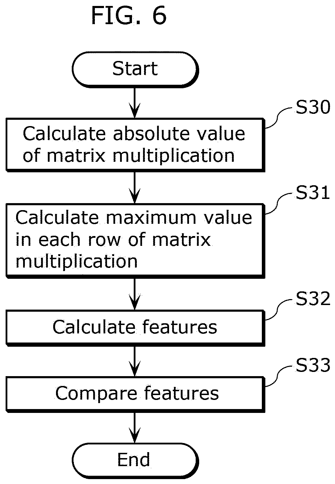

FIG. 6 is a flowchart showing a second example of the detailed operations of the estimation device according to Embodiment 1.
[FIG. 7]

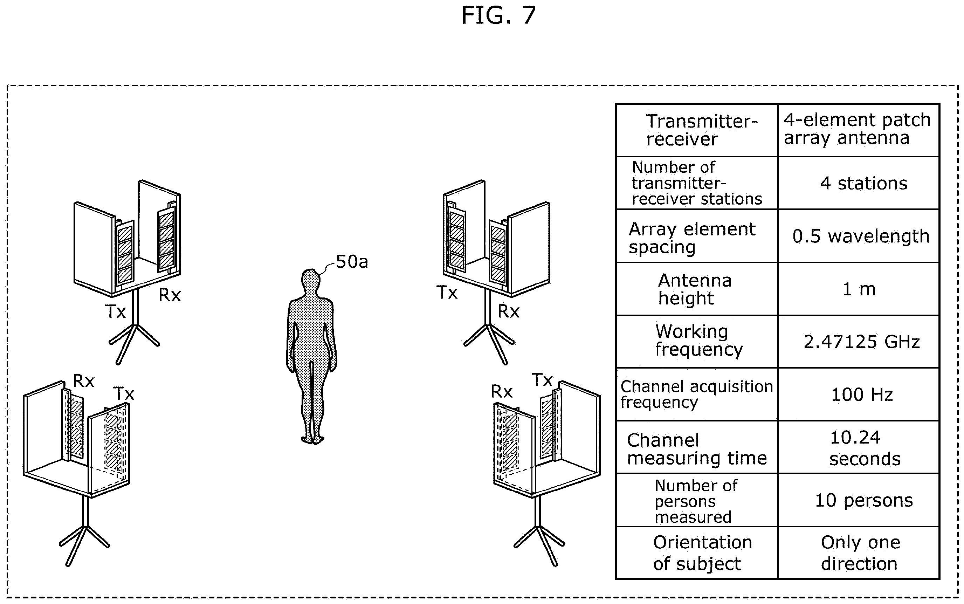

FIG. 7 is a diagram showing an environment in which the estimation device according to Embodiment 1 is used in a test.
[FIG. 8]

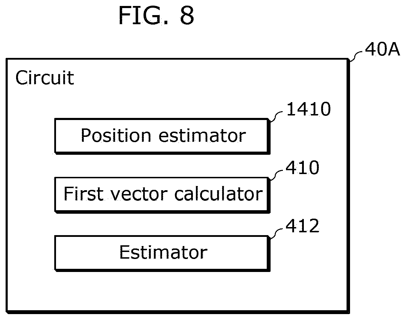

FIG. 8 is a schematic diagram showing one example of a detailed configuration of a circuit according to Embodiment 2.
[FIG. 9]

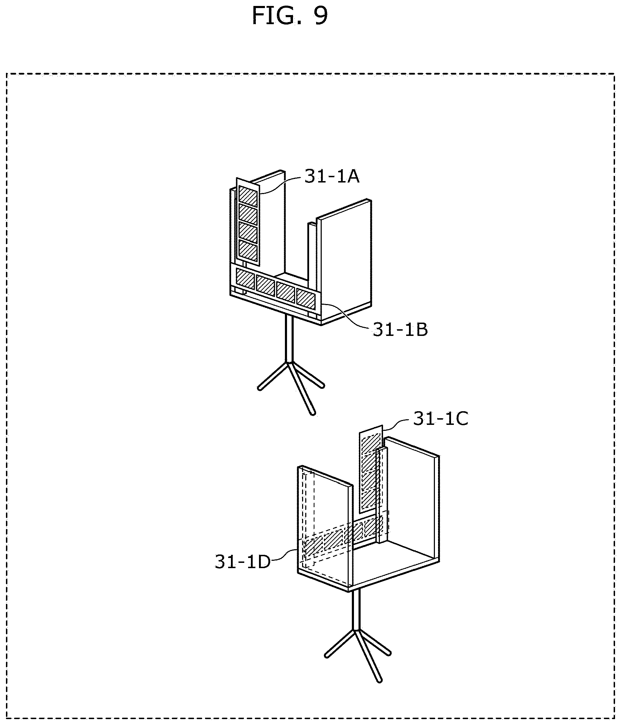

FIG. 9 is a diagram showing an exemplary arrangement of antenna elements according to Embodiment 2.
[FIG. 10]

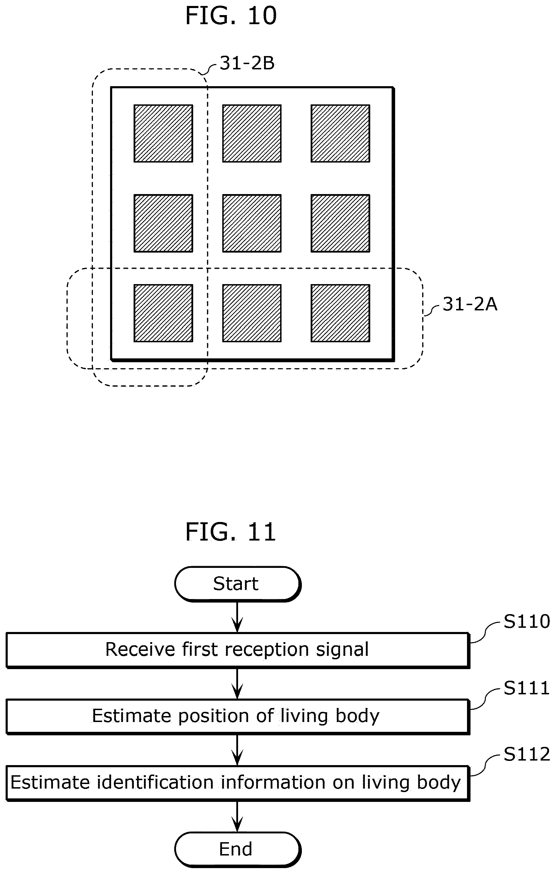

FIG. 10 is a diagram showing an exemplary arrangement of the antenna elements according to Embodiment 2.
[FIG. 11]
FIG. 11 is a flowchart showing one example of operations of an estimation device according to Embodiment 2.

[Description of Embodiments]

(Underlying Knowledge Forming Basis of the Present Disclosure)

PTLs 1 and 2 disclose devices for emitting electromagnetic waves to a person sitting in the driver seat of an automobile and measuring reflected waves of the electromagnetic waves received from the person. These devices measure heart beats and heart sound through computational processing performed on the result of the measurement and identify a living body by acquiring a time correlation of the measured heat beats or heart sound. The method disclosed in PTL 1 is, however, operable only in such limited environments that the positions of a subject and antennas can be specified in a confined space such as a driver seat as described above; in other words, there is a problem that the method disclosed in PTL 1 is not operable in environments other than the aforementioned environments.

In scenes such as daily life in indoor environments, there is demand for the ability to identify a living body in environments that antennas and a subject are distanced from each other and that there is flexibility in positional relationship between the antennas and the subject.

After many studies of this issue, the inventors of the present disclosure have found the following in order to identify a living body by using electromagnetic waves even if a subject and antennas are distanced from each other in indoor environments or the like.

To be more precise, they have found to install antenna elements around a room where a subject to be identified performs activities, transmit transmission waves from various directions, and receive reflected waves or scattered waves thereof at various directions so as to receive reception signals that have captured more features of a living body. Since the reception signals vary not a little depending on the distance between the living body and the antennas or the orientation or posture of the living body, a teaching signal is acquired while estimating the position or posture of the living body from the reception signals, and the aforementioned position or posture of the living body is stored as an identified position in order to identify the living body.

At this time, if the distance between the antennas and the subject is close enough as in the prior art example, the amplitudes of the reception signals are relatively large and it is possible to achieve the identification of an individual. However, if the identification of a living body using electromagnetic waves is conducted in environments such as indoor spaces where a subject and antennas are distanced from each other, the amplitudes of the reception signals become smaller due to attenuation caused by the distance and become difficult to be distinguished from noise floor. This deteriorates the accuracy of identification.

In view of this, the inventors of the present disclosure have found the fact that a target living body present in a region such as a living space can be identified accurately by calculating a frequency response matrix of reception signals, extracting frequency components that vary due to the activities of the living body, calculating a correlation matrix of the extracted frequency components for eigenvalue decomposition, and using eigen vectors as features to calculate coincidence with a teaching signal.

More specifically, in order to achieve the object described above, an estimation device according to one aspect of the present disclosure includes M transmitting antenna elements arranged around a predetermined range that includes a first living body, where M is an integer greater than or equal to 1, the M transmitting antenna elements each transmitting a first transmission signal to the predetermined range, N receivers arranged around the predetermined range and each including a receiving antenna element, where N is an integer greater than or equal to 3, the N receivers each receiving a first reception signal for a predetermined period of time by using the receiving antenna element, the first reception signal including a reflected signal obtained by the first transmission signal reflecting off the first living body, memory that stores a teaching signal that corresponds to $M \times N$ second reception signals obtained by causing each of the N receivers to receive a second reception signal in advance, the second reception signal including a reflected signal obtained by a second transmission signal reflecting off a second living body, the second transmission signal being transmitted from the M transmitting antenna elements to the second living body, a first vector calculator that calculates a teaching first vector from the teaching signal and a test first vector from $M \times N$ first reception signals obtained by causing each of the N receivers to receive the first reception signal, and an estimator that calculates a plurality of features from the teaching first vector and the test first vector and estimates identification information on the first living body by a predetermined method using the plurality of features calculated.

In this way, the estimation device calculates a plurality of features by using the teaching first vector and the test first vector obtained from the first reception signal and the teaching signal. The plurality of features as used herein may be calculated from, for example, a total sum of inner products of the first vectors or by machine learning using the k neighborhood method or any other method. Then, the identification information on the living body is estimated by determining whether the first living body is the same as the second living body, which is used to acquire the teaching signal, depending on whether the maximum feature among the plurality of features exceeds the threshold value. In this way, the estimation device uses the teaching first vector and the test first vector to calculate the plurality of features and, in other words, does not use temporal waveforms of the first reception signal and the teaching signal. If the temporal waveforms of the first reception signal and the teaching signal are used to calculate the plurality of features, a problem arises in that processing load and power consumption increase due to degradation in S/N ratio of components caused by the living body or high throughput required for sliding correlation calculation of the temporal waveforms. Since the estimation device uses the teaching first vector and the test first vector to calculate the plurality of features, it is possible to avoid the occurrence of those problems including deterioration in S/N ratio and an increase in processing load and power consumption. Accordingly, the estimation device is capable of identifying a living body by using electromagnetic waves even if the subject and the antennas are distanced from each other.

For example, the first vector calculator may calculate a teaching second matrix from the teaching signal, and calculates the teaching first vector by using decomposition of sorted elements of the teaching second matrix calculated, and calculate the test first vector by calculating a received second matrix from the first reception signal and using decomposition of sorted elements of the received second matrix calculated.

In this way, the first vector calculator calculates the first vector for each of the teaching signal and the first reception signal with ease by using decomposition of the sorted elements of the calculated second matrix and thereby estimates the identification information on the living body. Accordingly, the estimation device is capable of more easily identifying the living body by using electromagnetic waves even if the subject and the antennas are distanced from each other.

For example, the first vector calculator may calculate the teaching first vector that includes an infinite sequence as a component, the infinite sequence including a plurality of eigenvalues sorted in descending order, the plurality of eigenvalues being obtained by eigenvalue decomposition of the sorted elements of the teaching second matrix, and calculate the test first vector that includes an infinite sequence as a component, the infinite sequence including a plurality of eigenvalues sorted in descending order, the plurality of eigenvalues being obtained by eigenvalue decomposition of the sorted elements of the received second matrix.

In this way, the first vector calculator calculates the first vectors with ease by eigenvalue decomposition of the sorted elements of the second matrix and thereby estimates the identification information on the living body. Accordingly, the estimation device is capable of more easily identifying the living body by using electromagnetic waves even if the subject and the antenna are distanced from each other.

For example, the first vector calculator may calculate the teaching first vector that includes an infinite sequence as a component, the infinite sequence including a plurality of singular values sorted in descending order, the plurality of singular values being obtained by singular value decomposition of the sorted elements of the teaching second matrix, and calculate the test first vector that includes an infinite sequence as a component, the infinite sequence including a plurality of singular values sorted in descending order, the plurality of singular values being obtained by singular value decomposition of the sorted elements of the received second matrix.

In this way, the first vector calculator calculates the first vectors with ease by singular value decomposition of the sorted elements of the second matrix and thereby estimates the identification information on the living body. Accordingly, the estimation device is capable of more easily identifying the living body by using electromagnetic waves even if the subject and the antennas are distanced from each other.

For example, the estimator may identify the first living body by using a total sum of a predetermined number of features among the plurality of features.

In this way, the estimator identifies the first living body by using the total sum of a predetermined number of features among the plurality of features. At this time, using the fact that the probability of the first living body and the second living body being the same increases with increase in the aforementioned total sum of the features, the identification information can be estimated based on the sameness of the first living body and the second living body. Accordingly, the estimation device is capable of more easily identifying the living body by using electromagnetic waves even if the subject and the antennas are distanced from each other.

For example, at least either the M transmitting antenna elements or the N transmitting antenna elements may configure an array antenna for which directivity in a vertical direction is controllable.

In this way, the use of the array antenna for which directivity in the vertical direction is controllable enables grasping the features of the living body that is long in the vertical direction, and this contributes to appropriate estimation of the identification information. Accordingly, the estimation device is capable of more appropriately identifying the living body by using electromagnetic waves even if the subject and the antennas are distanced from each other.

For example, at least either the M transmitting antenna elements or the N transmitting antenna elements may configure an array antenna for which directivity in a horizontal direction is controllable. The estimation device may further include a position estimator that estimates a position of the first living body in accordance with the first reception signal and determines whether the position of the first living body is located within a predetermined region.

In this way, the use of the array antenna for which directivity in the horizontal direction is controllable enables further estimating the horizontal position of the living body. Accordingly, the estimation device is capable of estimating the position of the living body and identifying the living body by using electromagnetic waves even if the subject and the antennas are distanced from each other.

An estimation method according to one aspect of the present disclosure is an estimation method to be executed by an estimation device. The estimation device includes M transmitting antenna elements arranged around a predetermined range that includes a first living body, where M is an integer greater than or equal to 1, N receivers arranged around the predetermined range and each including a receiving antenna element, where N is an integer greater than or equal to three, and memory that stores a teaching signal that corresponds to M × N second reception signals obtained by causing each of the N receivers to receive a second reception signal in advance, the second reception signal including a reflected signal obtained by a second transmission signal reflecting off a second living body, the second transmission signal being transmitted from the M transmitting antenna elements to the second living body. The estimation method includes causing each of the M transmitting antenna elements to transmit a first transmission signal to the predetermined range, causing each of the N receivers to receive a first reception signal for a predetermined period of time by using the receiving antenna element, the first reception signal including a reflected signal obtained by the first transmission signal reflecting off the first living body, calculating a teaching first vector from the teaching signal and a test first vector from M × N first reception signals obtained by causing each of the N receivers to receive the first reception signal, and calculating a plurality of features from the teaching first vector and the test first vector and estimating identification information on the first living body by a predetermined method using the plurality of features calculated.

This estimation method achieves similar effects to those achieved by the estimation device described above.

These general or specific aspects of the present disclosure may be implemented as a system, a method, an integrated circuit, a computer program, or a computer-readable recording medium such as a CD-ROM, or may be implemented as an arbitrary combination of a system, a method, an integrated circuit, a computer program, or a recording medium.

Hereinafter, embodiments of the present disclosure will be described in detail with reference to the drawings. Note that each embodiment described below shows a specific preferred example of the present disclosure. Numerical values, shapes, materials, constituent elements, and the arrangement and connection of constituent elements, steps, the processing order of the steps, etc., shown in the following embodiments are mere examples and are not intended to limit the present disclosure. Among the constituent elements in the following embodiments, those that are not recited in any independent claim, which indicates the broadest concept of the present disclosure, are described as arbitrary constituent elements configuring a more preferable aspect. In the specification and the drawings, constituent elements that are substantially the same in functional configuration may be given the same reference signs, and redundant descriptions thereof shall be omitted.

[Embodiment 1]

The present embodiment describes an estimation device capable of identifying a living body by using electromagnetic waves even if a subject and antennas are distanced from each other.

[Configuration of Estimation Device 10A]

FIG. 1 is a schematic diagram showing one example of a configuration of estimation device 10A according to the present embodiment. FIG. 2 is a diagram showing one example of teaching signal 42 shown in FIG. 1. FIG. 3 is a schematic diagram showing one example of a detailed configuration of circuit 40 shown in FIG. 1.

Estimation device 10A shown in FIG. 1 is a device for estimating identification information on a living body and, in other words, a device for identifying a living body and generating identification information indicating the result of the identification.

Estimation device 10A includes M transmitting antenna elements (M is an integer greater than or equal to 1), N receivers (N is an integer greater than or equal to 3) each including a receiving antenna element, circuit 40, and memory 41.

The M transmitting antenna elements each transmit a transmission signal to predetermined range A1 that includes living body 50. The transmission signal is a high-frequency signal such as microwaves generated by a transmitter or the like. Living body 50 may, for example, be a human and this case is described as an example in the present disclosure, but the present disclosure is not limited thereto. Living body 50 is a subject to be estimated by estimation device 10A and is a living body to be identified (also referred to as a first living body). Predetermined range A1 is a space that has a predetermined range and includes living body 50. In other words, predetermined range A1 is a space used by estimation device 10A to estimate living body 50.

The identification of a living body includes the identification of an individual living body (i.e., individual) or the identification of the orientation of a living body. The orientation of a living body refers to a direction in which the living body is oriented or in other words represents a bearing on the orientation of the front of the living body and may be expressed by, for example, bearings such as north, south, east, and west or by which of the transmitting antenna elements or the receiving antennas the living body is oriented to.

For example, the M transmitting antenna elements each transmit a first transmission signal to predetermined range A1 that includes living body 50 as a subject to be identified. The M transmitting antenna elements also transmit a second transmission signal to predetermined range A1 that includes known living body 50 (also referred to as a second living body) in order to acquire a teaching signal. The transmitted first or second transmission signal is reflected by living body 50.

The N receivers each include a receiving antenna element and are arranged around predetermined range A1. The N receivers each use the receiving antenna element to receive a reception signal for a predetermined period of time, the reception signal including a reflected signal obtained by the transmission signal reflecting off living body 50. For example, each of the N receivers receives a first reception signal for a predetermined period of time by using the receiving antenna element, the first reception signal including a reflected signal obtained by the first transmission signal reflecting off the first living body. Each of the N receivers also receives a teaching signal for a period of time by using the receiving antenna element, the period of time being K times the predetermined period (K is greater than or equal to 2), the teaching signal being a second reception signal including a reflected signal obtained by the second transmission signal reflecting off the second living body.

In the present embodiment, estimation device 10A may include, for example, eight transmitter-receivers 30A to 30H, circuit 40, and memory 41 as shown in FIG. 1. That is, the M transmitting antenna elements and the N receivers may be configured as eight transmitter-receivers 30A, 30B, 30C, 30D, 30E, 30F, 30G, and 30H (30A to 30H). Note that the number of transmitter-receivers is not limited to eight.

The antenna elements included in the transmitter-receivers may configure an array antenna for which directivity is controllable in a direction perpendicular to a floor surface that is a horizontal surface (i.e., in the vertical direction). In this case, the effect of improving the accuracy of identifying living body 50 is achieved. In other words, at least either the M transmitting antenna elements or the N receiving antenna elements may configure an array antenna for which directivity in the vertical direction is controllable and, in this case, the effect of improving the accuracy of identifying living body 50 is achieved.

This is considered because living body 50 in an upright or sitting position is generally longer in the vertical direction than in the horizontal direction so that a larger number of features will appear in the vertical direction when living body 50 reflects or scatters radio waves.

Besides, features in the horizontal direction that appear when living body 50 reflects or scatters radio waves depends on the position of living body 50. Thus, the effect of improving robustness can be achieved by using features in the vertical direction obtained by the array antenna for which directivity in the vertical direction is controllable.

[Transmitter-receivers 30A to 30H]

In the present embodiment, eight transmitter-receivers 30A to 30H are arranged in positions around predetermined range A1 and transmit transmission signals to predetermined range A1 so as to receive reception signals that include reflected signals obtained by being reflected off living body 50. For example, eight transmitter-receivers 30A to 30H may be arranged in a circle at regular intervals and may be arranged in positions outside predetermined range A1.

As shown in FIG. 1, transmitter-receivers 30A to 30H each include one antenna element. Specifically, transmitter-receiver 30A includes antenna element 31A, and transmitter-receiver 30B includes antenna element 31B. The same applies to other transmitter-receivers 30C to 30H.

Transmitter-receivers 30A to 30H transmit transmission signals to predetermined range A1 by using antenna elements 31A to 31H. More specifically, transmitter-receivers 30A to 30H emit microwaves as the transmission signals to living body 50 by using antenna elements 31A to 31H. Note that transmitter-receivers 30A to 30H using antenna elements 31A to 31H may transmit unmodulated transmission signals, or may transmit transmission signals that have undergone modulation processing. In the case of transmitting transmission signals that have undergone modulation processing, transmitter-receivers 30A to 30H may further include a circuit for performing modulation processing.

Transmitter-receivers 30A to 30H also receive reception signals for a predetermined period of time by using antenna elements 31A to 31H, the reception signals including reflected signals obtained by the transmission signals reflecting off living body 50. Transmitter-receivers 30A to 30H output the obtained reception signals to circuit 40. Note that transmitter-receivers 30A to 30H each may include a circuit for processing a reception signal. In this case, each of transmitter-receivers 30A to 30H may convert the obtained reception signal into a low-frequency signal through frequency conversion processing. Transmitter-receivers 30A to 30H may further perform demodulation processing on the reception signals. Then, each of transmitter-receivers 30A to 30H may output a first reception signal obtained through the frequency conversion processing and/or the demodulation processing to circuit 40.

In the example shown in FIG. 1, the transmitters and the receivers are described as being configured as eight transmitter-receivers 30A to 30H each including four antenna elements that are common for transmission and reception, but the present disclosure is not limited thereto. The number of transmitter-receivers 30A to 30H are not limited to eight, and the estimation device may be configured by N transmitter-receivers (N is an integer greater than or equal to 3). As another alternative, a transmitter that includes M transmitting antenna elements (M is an integer greater than or equal to 1) and a receiver that includes N receiving antenna elements may be provided separately.

[Memory 41]

Memory 41 is an auxiliary storage device having a nonvolatile storage area and may, for example, be read only memory (ROM), flash memory, or a hard disk drive (HDD). For example, memory 41 may store information that is used in a variety of processing for operating estimation device 10A.

As shown in FIG. 1, memory 41 stores teaching signal 42. Teaching signal 42 is a signal waveform acquired in advance for the second living body, which is known living body 50 present in predetermined range A1. More specifically, teaching signal 42 corresponds to M × N second reception signals obtained by causing each of the N receivers to receive a second reception signal in advance, the second reception signal including a reflected signal obtained by the second transmission signal reflecting off the second living body, the second transmission signal being transmitted from each of the M transmitting antenna elements to the second living body. Teaching signal 42 as used herein may also correspond to M × N second reception signals obtained by causing each of the N receivers to receive the second reception signal in advance for a period of time that is K times the predetermined period of time (K is greater than or equal to two).

In the present embodiment, the M transmitting antenna elements and the N receivers are configured as eight transmitter-receivers 30A to 30H as shown in FIG. 1. One example of teaching signal 42 in this case will be described with reference to FIG. 2. Teaching signal 42 shown in FIG. 2 is one example of the reception signal received by one receiver during a measurement period.

Teaching signal 42 shown in FIG. 2 corresponds to a time response waveform of a plurality of reception signals received in advance by transmitter-receivers 30A to 30H, the reception signals including reflected signals obtained by the transmission signals reflecting off the surface of living body 50, the transmission signals being transmitted from antenna elements 31A to 31H to known living body 50 (second living body) present in predetermined range A1.

That is, teaching signal 42 shown in FIG. 2 corresponds to a plurality of reception signals received in advance by transmitter-receivers 30A to 30H for a measurement period, the reception signals including reflected signals. The measurement period as used herein refers to a period of time that is K times the above-described predetermined period (K is greater than or equal to 1). The measurement period may, for example, be 120 seconds, but the present disclosure is not limited thereto. The measurement period may be any length of time such as 3 seconds or 30 seconds as long as it is longer than or equal to the cardiac cycle of humans.

Note that teaching signal 42 may be acquired in advance for each of a plurality of known second living bodies. In this case, memory 41 may store a plurality of teaching signals 42 that correspond respectively to the plurality of known second living bodies in association with estimation information that is used to estimate a corresponding second living body.

Memory 41 may also store a plurality of teaching signals 42 that correspond to one second living body.

Moreover, teaching signal 42 may be acquired in advance for each of a plurality of postures (e.g., an upright position, a sitting position, and a lying position) of one second living body, or may be acquired in advance for each orientation of one living body. In this case, memory 41 may store teaching signals 42 in association with information on the posture or orientation of the second living body.

[Circuit 40]

Circuit 40 executes a variety of processing for operating estimation device 10A. For example, circuit 40 may be configured by a processor that executes control programs (e.g., central processing unit (CPU)) and a volatile storage area used as a work area during execution of the control programs (main memory). This storage area may, for example, be random access memory (RAM).

Circuit 40 temporarily stores the first reception signal acquired from each of the N receivers in the storage area for a predetermined period of time. Circuit 40 may temporarily store the phases and amplitudes of the first reception signals in the storage area for a predetermined period of time. In the present embodiment, circuit 40 temporarily stores the reception signals acquired from transmitter-receivers 30A to 30H in the storage area for a predetermined period of time.

Note that circuit 40 may be configured as a dedicated circuit for performing a variety of processing for operating estimation device 10A. That is, circuit 40 may be a circuit that performs software processing or a circuit that performs hardware processing. Circuit 40 may further include a nonvolatile storage area.

Next, a functional configuration of circuit 40 will be described.

Circuit 40 includes first vector calculator 410 and estimator 412 as shown in FIG. 3.

[First Vector Calculator 410]

More specifically, firstly, first vector calculator 410 calculates propagation channel matrices H(t) for the reception signals stored in the storage area of circuit 40 and for teaching signal 42 stored in memory 41 by using the reception signals and teaching signal 42.

The calculation of propagation channel matrices H(t) will be described later in detail. Here, more generally, a description is given of a method of calculating propagation channel matrices H(t) by using $K_R$ receivers (which correspond to the receivers described above) and $K_T$ transmitters (which correspond to the transmitters described above). The $K_R$ receivers each include $M_r$ receiving antenna elements, and the $K_T$ transmitters each include $M_t$ transmitting antenna elements. In this case, when a multiple-input and multiple-output (MIMO) array antenna including the aforementioned receiving antenna elements and the aforementioned transmitting antenna elements is arranged around living body 50, propagation channel matrices H(t) to be obtained are expressed by (Expression 1) and (Expression 2) below.

[Math. 1]

$$H(t) = \begin{pmatrix} H^{(11)}(t) & \cdots & H^{(1K_T)}(t) \\ \vdots & \ddots & \vdots \\ H^{(K_R1)}(t) & \cdots & H^{(K_RK_T)}(t) \end{pmatrix} \qquad \text{(Expression 1)}$$

[Math. 2]

$$H^{(k_Rk_T)}(t) = \begin{pmatrix} h_{11}^{(k_Rk_T)}(t) & \cdots & h_{1M_t}^{(k_Rk_T)}(t) \\ \vdots & \ddots & \vdots \\ h_{M_r1}^{(k_Rk_T)}(t) & \cdots & h_{M_rM_t}^{(k_Rk_T)}(t) \end{pmatrix} \qquad \text{(Expression 2)}$$

In (Expression 1) and (Expression 2), $K_R$ represents the number of receivers and $K_T$ represents the number of transmitters. Also, $k_R$ represents the receiver number and $k_T$ represents the transmitter number. $M_r$ represents the number of antenna elements included in each receiver and $M_t$ represents the number of antenna elements included in each transmitter. Also, $m_r$ represents the antenna element number of each antenna element included in each receiver and $m_t$ represents the antenna element number of each antenna element included in each transmitter.

[Math. 3]

$$h_{m_rm_t}^{(k_Rk_T)}$$

represents the complex channel response from the $m_t$-th antenna of transmitter $k_T$ to the $m_r$-th antenna of receiver $k_R$, and t represents the observation time.

Although the complex channel response is defined for each combination of a plurality of transmitters and receivers in the present embodiment, the complex channel response may be expressed by one matrix by regarding a plurality of transmitters as one large transmitter and a plurality of receivers as one large receiver.

Next, first vector calculator 410 calculates frequency response matrix F(ω) of propagation channel matrices H(t). Frequency response matrix F(ω) is expressed by (Expression 3).

[Math. 4]

$$F^{(k_Rk_T)}(\omega) = \begin{pmatrix} f_{11}^{(k_Rk_T)}(\omega) & \cdots & f_{1M_t}^{(k_Rk_T)}(\omega) \\ \vdots & \ddots & \vdots \\ f_{M_r1}^{(k_Rk_T)}(\omega) & \cdots & f_{M_rM_t}^{(k_Rk_T)}(\omega) \end{pmatrix} \qquad \text{(Expression 3)}$$

Here, ω represents the frequency range corresponding to living body 50 (in other words, the frequency range corresponding to frequency components that vary due to the activities of living body 50). Note that the frequency response matrix is obtained by Fourier transform or by calculation of a time difference between the propagation channel matrices.

Next, first vector calculator 410 sorts (more specifically, vectorizes) propagation channel matrices H(t) and frequency response matrix F(ω) as follows.

[Math. 5]

$$vec\{\boldsymbol{H}(t)\}、 \; vec\{\boldsymbol{F}(\omega)\}$$

[Math. 6]

$$vec\{\boldsymbol{H}(t)\}、 \; vec\{\boldsymbol{F}(\omega)\}$$

are expressed respectively by (Expression 4) and (Expression 5), where T represents the transpose.
[Math. 7]

$$vec\{\boldsymbol{H}^{(k_R k_T)}(\omega)\} = [h_{11}^{(k_R k_T)}(\omega) \cdot \cdot \cdot h_{M_r 1}^{(k_R k_T)}(\omega), \cdot \cdot \cdot, \; h_{1M_t}^{(k_R k_T)}(\omega) \cdot \cdot \cdot$$

$$h_{M_r M_t}^{(k_R k_T)}(\omega)]^T \qquad \text{(Expression 4)}$$

[Math. 8]

$$vec\{\boldsymbol{F}^{(k_R k_T)}(\omega)\} = [f_{11}^{(k_R k_T)}(\omega) \cdot \cdot \cdot f_{M_r 1}^{(k_R k_T)}(\omega), \cdot \cdot \cdot, \; f_{1M_t}^{(k_R k_T)}(\omega) \cdot \cdot \cdot$$

$$f_{M_r M_t}^{(k_R k_T)}(\omega)]^T \qquad \text{(Expression 5)}$$

Next, first vector calculator 410 calculates second matrix R from the vector matrix below.

[Math. 9]

$$vec\{\boldsymbol{F}(\omega)\}$$

Second matrix R is expressed by (Expression 6), where

[Math. 10]

$$E[\{\} \cdot \{\}]$$

represents the ensemble average.
[Math. 11]

$$\boldsymbol{R}^{(k_R k_T)} = E[vec\{\boldsymbol{F}^{(k_R k_T)}(\omega)\}vec\{(\boldsymbol{F}^{(k_R k_T)})^H(\omega)\}] \qquad \text{(Expression 6)}$$

Next, first vector calculator 410 obtains eigen vectors (which correspond to first vectors) below:

[Math. 12]

$$U^{(k_R k_T)}$$

and diagonal components of the eigenvalues below:

[Math. 13]

$$\Lambda^{(k_R k_T)}$$

by eigenvalue decomposition of second matrix R. The eigenvalue decomposition is expressed by (Expression 7), (Expression 8), and (Expression 9), where H represents the complex conjugate transpose.

[Math. 14]

$$R^{(k_R k_T)} = U^{(k_R k_T)} \Lambda^{(k_R k_T)} (U^{(k_R k_T)})^H \qquad \text{(Expression 7)}$$

[Math. 15]

$$\Lambda^{(k_R k_T)} = \mathrm{diag}\left[\lambda_1^{(k_R k_T)}, \cdots, \lambda_l^{(k_R k_T)}, \cdots, \lambda_{M_r M_t}^{(k_R k_T)}\right] \qquad \text{(Expression 8)}$$

$$\left( \therefore \lambda_1 \geq \lambda_2 \geq \cdots, \lambda_L > \lambda_{L+1} = \cdots = \lambda_{M_R M_T} \right)$$

[Math. 16]

$$U_1^{(k_R k_T)} = \left[u_1^{(k_R k_T)} \cdots, u_l^{(k_R k_T)}, \cdots, u_{M_r M_t}^{(k_R k_T)}\right] \qquad \text{(Expression 9)}$$

Here, l is the integer greater than or equal to 1 and less than $M_r$ by $M_t$.

In this way, first vector calculator 410 calculates the first vectors for both of the first reception signals and teaching signal 42. The first vectors calculated for the first reception signals are also referred to as test first vectors, and the first vector calculated for teaching signal 42 is also referred to as a teaching first vector.

Note that memory 41 may store, instead of teaching signals 42, the first vectors calculated based on teaching signals 42 by first vector calculator 410.

[Estimator 412]

Estimator 412 performs estimation by evaluating coincidences between the first vectors below calculated based on the reception signals ([Math. 17]) and the first vector calculated from teaching signal 42 ([Math. 18]).

[Math. 17]

$$U_T^{(k_R k_T)}$$

[Math. 18]

$$U_D^{(k_R k_T)}$$

The present embodiment describes a method of performing estimation by using features obtained from the total sum of inner products.

First, estimator 412 calculates the absolute value of a matrix multiplication of the first vectors ([Math. 19] and [Math. 20]) replaced by complex conjugate transpose as indicated by (Expression 10).

$$[\text{Math. } 19]$$
$$\boldsymbol{U}_T{}^{(k_R k_T)}$$

$$[\text{Math. } 20]$$
$$\boldsymbol{U}_D{}^{(k_R k_T)}$$

[Math. 21]

$$\left| (\boldsymbol{U}_T^{(k_R k_T)})^H \boldsymbol{U}_D^{(k_R k_T)} \right| = \left\| \begin{bmatrix} \left(\boldsymbol{u}_{T_1}^{(k_R k_T)}\right)^H \\ \left(\boldsymbol{u}_{T_l}^{(k_R k_T)}\right)^H \\ \left(\boldsymbol{u}_{T_{M_r M_t}}^{(k_R k_T)}\right)^H \end{bmatrix} \left[ \boldsymbol{u}_{D_1}^{(k_R k_T)}, \cdots, \boldsymbol{u}_{D_l}^{(k_R k_T)}, \cdots, \boldsymbol{u}_{D_{M_r M_t}}^{(k_R k_T)} \right] \right\|$$

$$= \left| \begin{matrix} \left(\boldsymbol{u}_{T_1}^{(k_R k_T)}\right)^H \boldsymbol{u}_{D_1}^{(k_R k_T)} & \cdots & \left(\boldsymbol{u}_{T_1}^{(k_R k_T)}\right)^H \boldsymbol{u}_{D_l}^{(k_R k_T)} & \cdots & \left(\boldsymbol{u}_{T_1}^{(k_R k_T)}\right)^H \boldsymbol{u}_{D_{M_r M_t}}^{(k_R k_T)} \\ \vdots & \ddots & \vdots & & \vdots \\ \left(\boldsymbol{u}_{T_l}^{(k_R k_T)}\right)^H \boldsymbol{u}_{D_1}^{(k_R k_T)} & \cdots & \left(\boldsymbol{u}_{T_l}^{(k_R k_T)}\right)^H \boldsymbol{u}_{D_l}^{(k_R k_T)} & \cdots & \left(\boldsymbol{u}_{T_l}^{(k_R k_T)}\right)^H \boldsymbol{u}_{D_{M_r M_t}}^{(k_R k_T)} \\ \vdots & & \vdots & \ddots & \vdots \\ \left(\boldsymbol{u}_{T_{M_r M_t}}^{(k_R k_T)}\right)^H \boldsymbol{u}_{D_1}^{(k_R k_T)} & \cdots & \left(\boldsymbol{u}_{T_{M_r M_t}}^{(k_R k_T)}\right)^H \boldsymbol{u}_{D_l}^{(k_R k_T)} & \cdots & \left(\boldsymbol{u}_{T_{M_r M_t}}^{(k_R k_T)}\right)^H \boldsymbol{u}_{D_{M_r M_t}}^{(k_R k_T)} \end{matrix} \right|$$

$$(\text{Expression } 10)$$

Then, estimator 412 obtains and extracts a maximum value in each row of the matrix obtained from (Expression 10). The extracted maximum value in the first row is expressed by:

$$[\text{Math. } 22]$$
$$\left| \left(\boldsymbol{u}_{T_l}^{(k_R k_T)}\right)^H \boldsymbol{u}_{D_{l_{max}}}^{(k_R k_T)} \right|$$

Next, estimator 412 obtains feature S from (Expression 11).
[Math. 23]

$$S = \sum_{k_R=1}^{K_R} \sum_{k_T=1}^{K_T} \sum_{l=1}^{L} \left| \left(\boldsymbol{u}_{T_l}^{(k_R k_T)}\right)^H \boldsymbol{u}_{D_{l_{max}}}^{(k_R k_T)} \right| \qquad (\text{Expression } 11)$$

Here, L represents the number of eigen vectors to be used. When the first vectors are sorted in advance in descending order of the eigen values, computational complexity can be reduced by reducing L. Estimator 412 performs the processing expressed by (Expression 11) for each teaching signal 42 stored in memory 41 and estimates one teaching signal 42 that has maximum feature S as a correct living body.

In a predetermined combination of $k_R$ and $k_T$ in (Expression 11), estimator 412 may zero the following value:

[Math. 24]

$$\left| \left( u_{T_l}^{(k_R k_T)} \right)^H u_{D_{l_{max}}}^{(k_R k_T)} \right|$$

or may subtract this value at a predetermined ratio. In this way, estimator 412 is capable of excluding, for example, the results of reception in which large direct waves are transmitted and received between the opposed transmitters and receivers and thus result in adverse effects.

In the case where each of features S calculated from a plurality of teaching signals 42 does not exceed a preset threshold value, estimator 412 may output information indicating that there is no corresponding living body as the result of estimation. In the case where there are a plurality of features S that exceed the aforementioned threshold value, estimator 412 may output a plurality of pieces of identification information on living body 50 as the results of estimation.

Although the present embodiment has described the case of estimation using one reception signal, the accuracy of estimation can be improved if estimator 412 performs estimation multiple times by observing a plurality of reception signals or by dividing a reception signal into a plurality of signals to obtain a plurality of first vectors. More specifically, if a mode of a plurality of results of estimating the living body is used or an estimation result that exhibits a highest average or maximum value of features S is adopted among a plurality of results of estimating the living body, the number of errors in estimation can be reduced as compared to the case of estimation using only one reception signal.

Although estimator 412 according to the present embodiment employs features using a matrix multiplication of the first vectors, the present disclosure is not limited thereto as long as the method allows evaluation of coincidence of the first vectors and, for example, the coincidence may be evaluated by machine learning such as the k neighborhood method. For example, estimator 412 is capable of performing learning using the k neighborhood method by standardizing the following first vector:

[Math. 25]

$$u_l^{(k_R k_T)}$$

by using the own first element so as to calculate:

[Math. 26]

$$u_l^{(k_R k_T)'}$$

and dividing this result into the real part and the imaginary part to obtain $M_r$ by $M_t$ by 2 features.

At this time, if teaching signal 42 is the signal acquired when the second living body is oriented in various directions, estimator 412 is capable of identifying the orientation of living body 50. If teaching signal 42 is the signal acquired from a plurality of different living bodies when the orientation of the second living body is fixed, estimator 412 is capable of identifying living body 50 (i.e., determining which one of the above living bodies is living body 50).

In this way, estimation device 10A shown in FIG. 1 is capable of identifying living body 50 by processing the reception signals received from transmitter-receivers 30A to 30H in circuit 40.

[Operations of Estimation Device 10A]

Next, operations of estimation device 10A configured as described above will be described.

FIG. 4 is a flowchart showing one example of the operations of estimation device 10A according to the present embodiment. FIG. 5 is a flowchart showing detailed operations of estimation device 10A according to the present embodiment (operations included in step S11 in FIG. 4). FIG. 6 is a flowchart showing detailed operations of estimation device 10A according to the embodiment (operations included in step S12 in FIG. 4).

First, estimation device 10A transmits M transmission signals and receives N reception signals (S10). The N reception signals correspond to the first reception signals received by the N receivers.

More specifically, estimation device 10A transmits first transmission signals to predetermined range A1 that includes a first living body by using M transmitting antenna elements (transmitter $K_T$ × the number of transmitting antenna elements $M_t$). Then, estimation device 10A causes each of the N receivers (receivers $K_R$ × the number of receiving antennas $M_r$) to receive a first reception signal for a predetermined period of time by using the receiving antenna elements, the first reception signal including a reflection signal obtained by a first transmission signal reflecting off the first living body.

In the present embodiment, transmitter-receivers 30A to 30H cause antenna elements 31A to 31H to transmit transmission signals to predetermined range A1 while the first living body, which is living body 50 to be estimated, is arranged within predetermined range A1. Then, transmitter-receivers 30A to 30H receive first reception signals for a predetermined period of time by using antenna elements 31A to 31H, the first reception signals being obtained by the first transmission signals reflecting off the first living body.

Next, estimation device 10A calculates first vectors from the first reception signals acquired in step S10 and teaching signal 42 stored in memory 41 (S11).

Processing included in step S11 will be described with reference to FIG. 5.

First, estimation device 10A reads out teaching signal 42 from memory 41, the teaching signal corresponding to M × N second reception signals obtained by causing the N receivers to receive reflected signals in advance, the reflected signals being obtained by the second transmission signals reflecting off the second living body, the second transmission signals being transmitted from the M transmitting antenna elements to the second living body which is a known living body. Then, estimation device 10A calculates frequency response matrices by Fourier transform of the first reception signals acquired in step S10 and the teaching signal read out from memory 41 or by a time difference therebetween (S21). Then, estimation device 10A vectorizes each frequency response matrix and calculates a correlation matrix thereof as a second matrix (S22). Thereafter, estimation device 10A conducts eigenvalue decomposition of the second matrix (S23) and calculates the first vectors by sorting the eigenvalues in descending order of diagonal elements (S24).

In the present embodiment, circuit 40 calculates the first vectors for both of the first reception signals and teaching signal 42. The first vectors calculated for the first reception signals are also referred to as test first vectors, and the first vector calculated for teaching signal 42 is also referred to as a teaching first vector.

Although the eigenvalue decomposition is used in step S23, instead of this, singular value decomposition of the second matrix may be conducted and a singular vector (which corresponds to the first vector) may be calculated based on the singular values in step S24. At this time, the singular vector may be obtained by sorting the singular values in descending order. Note that the decomposition of the matrix or the decomposition of the matrix elements includes at least eigenvalue decomposition or singular value decomposition.

Referring back to FIG. 4, next, estimation device 10A estimates identification information on the living body by using the first vectors for the first reception signals and teaching signal 42 (S12).

Processing included in step S12 will be described with reference to FIG. 6.

First, estimation device 10A calculates the absolute value of a matrix multiplication of the first vectors for the first reception signals and teaching signal 42 (S30). Then, estimation device 10A calculates a maximum value in each row of the matrix multiplication calculated in step S30 (S31). Then, estimation device 10A adds the maximum values calculated in step S31 in the column direction, performs similar calculation for every combination of transmitters and receivers, and performs addition or multiplication of all values so as to calculate features (S32). Then, estimation device 10A compares a plurality of features calculated from each of a plurality of teaching signals 42 stored in memory 41 in step S32 and outputs identification information on the living body that is related to teaching signal 42 exhibiting a maximum feature as estimated identification information on living body 50 (S33).

In step S33, if all of the features do not exceed a preset threshold value or if a difference or ratio between the maximum feature and the second maximum feature is less than or equal to a fixed value, estimation device 10A may output information indicating that there is no corresponding living body as the result of estimation. If there are a plurality of features that exceed the threshold value, a plurality of pieces of identification information on living body 50 may be output as the results of estimation.

[Specific Example of Configuration]

FIG. 7 is a diagram showing one example of the environment used in an estimation test conducted by estimation device 10A according to the embodiment.

As shown in FIG. 7, the estimation test uses four transmitters-receivers that correspond to transmitter-receivers 30B, 30D, 30F, and 30H (see FIG. 1). Note that the receivers and transmitters may be configured by eight transmitter-receivers as shown in FIG. 1, or may be configured by four transmitter-receivers or any number of transmitters and receivers as long as the number is two or more.

The four transmitter-receivers are arranged at the vertices of a square having 4-cm edges and centered on subject 50a. Subject 50a corresponds to living body 50 to be estimated in the estimation test, i.e., the first living body.

As the receiving antenna elements and the transmitting antenna elements that correspond to antenna elements 31B, 31D, 31F, and 31H, square patch antennas are used each including four elements arranged in the vertical direction. More specifically, the four receiving antenna elements included in the four transmitter-receivers are each a square patch antenna and installed at a height of 0.9 m from the floor surface. The four transmitting antenna elements included in the four transmitter-receivers are each arranged in a place spaced in the horizontal direction by one wavelength of microwaves of the corresponding receiving antenna element. Here, the transmitting antennas and the receiving antennas may be shared, or may be provided separately.

[Advantageous Effects]

In the environment used in the test shown in FIG. 7, estimation device 10A transmits transmission waves from the antenna elements of the transmitters or the receivers, which are installed in, for example, four places around living body 50, and receives reception signals. Then, estimation device 10A calculates the second matrix from the teaching signal stored in memory 41 and the reception signals received from subject 50a serving as living body 50 to be identified, and calculates the first vectors by eigenvalue decomposition of the calculated second matrix. Since a first vectors with a larger eigenvalue includes a larger number of components of the living body, estimation device 10A is capable of increasing the S/N ratio of the components of the living body by narrowing down eigenvalues to be used.

As described above, estimation device 10A according to the present embodiment emphasizes components caused by fluctuations of the living body among the first reception signals and the teaching signal by frequency response conversion, the first reception signals being measurement signals obtained from the receiving antenna elements installed around the first living body, and in this state can obtain features by calculating the first vectors by eigenvalue decomposition of the correlation matrix and calculating the inner products or the matrix multiplication. This allows estimation device 10A to identify the living body even under such conditions that the distance between the living body and the antennas is relatively large and the S/N ratio of the components of the living body is relatively low. Accordingly, estimation device 10A is capable of identifying the living body by estimating that the first living body is the same as the second living body, which is used to acquire the teaching signals, depending on whether the maximum value among a plurality of correlation coefficients exceeds a threshold value.

Estimation device 10A according to the present embodiment is also capable of estimating the identification information on living body 50 such as a human by using radio signals such as microwaves. That is, estimation device 10A according to the present embodiment is capable of estimating the identification information on living body 50 such as a human without the need for image analysis of images captured by a camera or any other device. This may contribute to human identification under the protection of human privacy.

[Embodiment 2]

The present embodiment describes an estimation device that combines the estimation of the position of a living body and the identification of the living body described in Embodiment 1. The estimation device according to the present embodiment is identical in configuration to estimation device 10A according to Embodiment 1 shown in FIG. 1, and thus descriptions of constituent elements that are identical in configuration shall be omitted.

The estimation device according to the present embodiment includes circuit 40A shown in FIG. 8, instead of circuit 40 of estimation device 10A according to Embodiment 1. Circuit 40A includes position estimator 1410, first vector calculator 410, and estimator 412.

[Position Estimator 1410]

Position estimator 1410 estimates the position of living body 50 present in predetermined range A1 in accordance with first reception signals that are a propagation channel matrix obtained in the same manner as in Embodiment 1. The method of estimating the position of living body 50 from the propagation channel matrix is disclosed in, for example, PTL 6.

Note that at least either the transmitting antenna elements or the receiving antenna elements may configure an array antenna for which directivity in the horizontal direction is controllable. In that case, position estimator 1410 uses, as the first reception signals, reception signals that are either signals transmitted from a transmitting array antenna for which directivity in the horizontal direction is controllable or signals transmitted from a receiving array antenna for which directivity in the horizontal direction is controllable so as to estimate the position of the first living body and determine whether the position of the first living body is located within a predetermined region.

For example, antenna elements 31A to 31H included in the estimation device according to the present embodiment configure an array antenna for which directivity in the horizontal direction is controllable. In order to identify living body 50 as described in Embodiment 1, if the directivity for antenna elements 31A to 31H in the vertical direction is also controllable it is possible to achieve the effect of improving the accuracy of estimation of living body 50. FIGS. 9 and 10 show examples of such characterized antenna elements.

FIG. 9 shows a configuration that combines an array antenna in which antenna elements are aligned in the longitudinal direction and an array antenna in which antenna elements are aligned in the lateral direction (namely, antenna arrays 31-1A, 31-1B, 31-1C, and 31-1D).

FIG. 10 shows antenna array 31-2A that uses a plurality of antenna elements aligned in a straight line in the lateral direction and antenna array 31-2B that uses a plurality of antenna elements aligned in a straight line in the longitudinal direction, among antenna elements arranged in a rectangle (or grid).

Among the antenna arrays shown in FIGS. 9 and 10, signals received by the array antennas in which antenna elements are aligned in the longitudinal direction (also referred to as longitudinal antennas; namely, antenna arrays 31-1A and 31-1C (see FIG. 9) and antenna array 31-2B (see FIG. 10)) may be used properly in the identification of a living body.

Among the antenna arrays shown in FIGS. 9 and 10, signals received by the array antennas in which antenna elements are aligned in the lateral direction (also referred to as lateral arrays; namely antenna arrays 31-1B and 31-1D (see FIG. 9) and antenna array 31-2A (see FIG. 10)) may be used properly in the estimation of the position of a living body.

Position estimator 1410 determines, based on such estimated position, whether it is possible to start the identification of a living body by a predetermined method. More specifically, position estimator 1410 starts the identification of a living body when the distance between the estimated position of a living body and either a preset position or the position stored in memory 41 is less than or equal to a predetermined threshold value.

The processing for identifying a living body is similar to that described in Embodiment 1 and thus a detailed description thereof shall be omitted.

Note that first vector calculator 410 may calculate the first vectors by using the first reception signals used by position estimator 1410, or may observe new first reception signals.

[Operations of Estimation Device]

Next, operations of the estimation device configured as described above will be described.

FIG. 11 is a flowchart showing one example of the operations of the estimation device according to the present embodiment.

First, the estimation device transmits M transmission signals and receives N reception signals (S110). The N reception signals correspond to the first reception signals received by the N receivers.

Next, the estimation device estimates the position of the living body on the basis of the reception signals received in step S110 and determines whether it is possible to start the identification of the living body (S111). More specifically, the estimation device determines whether the distance between the estimated position of the living body and either the preset position or the position stored in advance is less than or equal to a predetermined threshold value, and determines that it is possible to start the identification of the living body when the above distance is determined to be less than or equal to the above predetermined threshold value.

Next, the estimation device estimates identification information on the living body by a method similar to that used by estimation device 10A (S112).

[Advantageous Effects]

The use of the estimation device according to the present embodiment enables the use of teaching data that is measured when the living body is located in the same position as in the case of test data. This reduces the influence of the position of the living body and increases the accuracy of identification of the living body.

[Industrial Applicability]

The present disclosure is applicable to the estimation device and the estimation method for estimating a living body by using radio signals and, in particular, applicable to the estimation device and the estimation method that are installed in equipment such as home-use equipment that performs control depending on the living body or a monitoring device that detects ingress of a living body.

[Reference Signs List]

10A estimation device
30A, 30B, 30C, 30D, 30E, 30F, 30G, 30H transmitter-receiver
31A, 31B, 31C, 31D, 31E, 31F, 31G, 31H antenna element
31-1A, 31-1B, 31-1C, 31-1D, 31-2A, 31-2B antenna array
40, 40A circuit
41 memory

42 teaching signal
50 living body
50a subject
410 first vector calculator
412 estimator
1410 position estimator

**Claims**

1. An estimation device comprising:

   M transmitting antenna elements arranged around a predetermined range that includes a first living body, where M is an integer greater than or equal to 1, the M transmitting antenna elements each transmitting a first transmission signal to the predetermined range;
   N receivers arranged around the predetermined range and each including a receiving antenna element, where N is an integer greater than or equal to 3, the N receivers each receiving a first reception signal for a predetermined period of time by using the receiving antenna element, the first reception signal including a reflected signal obtained by the first transmission signal reflecting off the first living body;
   memory that stores a teaching signal that corresponds to M × N second reception signals obtained by causing each of the N receivers to receive a second reception signal in advance, the second reception signal including a reflected signal obtained by a second transmission signal reflecting off a second living body, the second transmission signal being transmitted from the M transmitting antenna elements to the second living body;
   a first vector calculator that calculates a teaching first vector from the teaching signal and a test first vector from M × N first reception signals obtained by causing each of the N receivers to receive the first reception signal; and
   an estimator that calculates a plurality of features from the teaching first vector and the test first vector and estimates identification information on the first living body by a predetermined method using the plurality of features calculated.

2. The estimation device according to claim 1,
   wherein the first vector calculator:

   calculates a teaching second matrix from the teaching signal, and calculates the teaching first vector by using decomposition of sorted elements of the teaching second matrix calculated; and
   calculates the test first vector by calculating a received second matrix from the first reception signal and using decomposition of sorted elements of the received second matrix calculated.

3. The estimation device according to claim 2,
   wherein the first vector calculator:

   calculates the teaching first vector that includes an infinite sequence as a component, the infinite sequence including a plurality of eigenvalues sorted in descending order, the plurality of eigenvalues being obtained by eigenvalue decomposition of the sorted elements of the teaching second matrix; and
   calculates the test first vector that includes an infinite sequence as a component, the infinite sequence including a plurality of eigenvalues sorted in descending order, the plurality of eigenvalues being obtained by eigenvalue decomposition of the sorted elements of the received second matrix.

4. The estimation device according to claim 2,
   wherein the first vector calculator:

   calculates the teaching first vector that includes an infinite sequence as a component, the infinite sequence including a plurality of singular values sorted in descending order, the plurality of singular values being obtained by singular value decomposition of the sorted elements of the teaching second matrix; and
   calculates the test first vector that includes an infinite sequence as a component, the infinite sequence including a plurality of singular values sorted in descending order, the plurality of singular values being obtained by singular value decomposition of the sorted elements of the received second matrix.

5. The estimation device according to any one of claims 1 to 4,

wherein the estimator identifies the first living body by using a total sum of a predetermined number of features among the plurality of features.

6. The estimation device according to any one of claims 1 to 5,
   wherein at least either the M transmitting antenna elements or the N transmitting antenna elements configure an array antenna for which directivity in a vertical direction is controllable.

7. The estimation device according to any one of claims 1 to 6,

   wherein at least either the M transmitting antenna elements or the N transmitting antenna elements configure an array antenna for which directivity in a horizontal direction is controllable,
   the estimation device further comprising:
   a position estimator that estimates a position of the first living body in accordance with the first reception signal and determines whether the position of the first living body is located within a predetermined region.

8. An estimation method to be executed by an estimation device,
   the estimation device including:

   M transmitting antenna elements arranged around a predetermined range that includes a first living body, where M is an integer greater than or equal to 1;
   N receivers arranged around the predetermined range and each including a receiving antenna element, where N is an integer greater than or equal to three; and
   memory that stores a teaching signal that corresponds to M × N second reception signals obtained by causing each of the N receivers to receive a second reception signal in advance, the second reception signal including a reflected signal obtained by a second transmission signal reflecting off a second living body, the second transmission signal being transmitted from the M transmitting antenna elements to the second living body,
   the estimation method comprising:

   causing each of the M transmitting antenna elements to transmit a first transmission signal to the predetermined range;
   causing each of the N receivers to receive a first reception signal for a predetermined period of time by using the receiving antenna element, the first reception signal including a reflected signal obtained by the first transmission signal reflecting off the first living body;
   calculating a teaching first vector from the teaching signal and a test first vector from M × N first reception signals obtained by causing each of the N receivers to receive the first reception signal; and
   calculating a plurality of features from the teaching first vector and the test first vector and estimating identification information on the first living body by a predetermined method using the plurality of features calculated.

# FIG. 1

# FIG. 2

# FIG. 3

## FIG. 4

Start

Receive first reception signal — S10

Calculate first vector — S11

Estimate identification information on living body — S12

End

## FIG. 5

Start

Calculate frequency response matrix — S21

Calculate second matrix — S22

Decompose second matrix — S23

Calculate first vector — S24

End

# FIG. 6

```
                    ┌──────────────┐
                    │    Start     │
                    └──────────────┘
                           │
                           ▼                    ⌐S30
        ┌────────────────────────────────────┐
        │ Calculate absolute value           │
        │ of matrix multiplication           │
        └────────────────────────────────────┘
                           │
                           ▼                    ⌐S31
        ┌────────────────────────────────────┐
        │ Calculate maximum value            │
        │ in each row of matrix              │
        │ multiplication                     │
        └────────────────────────────────────┘
                           │
                           ▼                    ⌐S32
        ┌────────────────────────────────────┐
        │       Calculate features           │
        └────────────────────────────────────┘
                           │
                           ▼                    ⌐S33
        ┌────────────────────────────────────┐
        │       Compare features             │
        └────────────────────────────────────┘
                           │
                           ▼
                    ┌──────────────┐
                    │     End      │
                    └──────────────┘
```

## FIG. 7

| Transmitter-receiver | 4-element patch array antenna |
|---|---|
| Number of transmitter-receiver stations | 4 stations |
| Array element spacing | 0.5 wavelength |
| Antenna height | 1 m |
| Working frequency | 2.47125 GHz |
| Channel acquisition frequency | 100 Hz |
| Channel measuring time | 10.24 seconds |
| Number of persons measured | 10 persons |
| Orientation of subject | Only one direction |

EP 4 365 622 A1

# FIG. 8

40A

Circuit

Position estimator — 1410

First vector calculator — 410

Estimator — 412

# FIG. 9

31-1A

31-1B

31-1C

31-1D

# FIG. 10

31-2B

31-2A

# FIG. 11

Start

Receive first reception signal —— S110

Estimate position of living body —— S111

Estimate identification information on living body —— S112

End

## EP 4 365 622 A1

| | | |
|---|---|---|
| **INTERNATIONAL SEARCH REPORT** | International application No. | |
| | **PCT/JP2022/025557** | |

**A. CLASSIFICATION OF SUBJECT MATTER**

*G01S 7/02*(2006.01)i; *G01S 13/50*(2006.01)i; *A61B 5/107*(2006.01)i; *A61B 5/11*(2006.01)i; *A61B 5/117*(2016.01)i
FI:  G01S7/02 216; G01S13/50; A61B5/11 110; A61B5/117 200; A61B5/107 300

According to International Patent Classification (IPC) or to both national classification and IPC

**B. FIELDS SEARCHED**

Minimum documentation searched (classification system followed by classification symbols)

G01S7/00-G01S7/42; G01S13/00-G01S13/95; A61B5/06-A61B5/22

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | 林哲平, 複数局MIMOレーダを用いた非拘束生体向き推定法, 電子情報通信学会2020年総合大会講演論文集 通信1, 03 March 2020, B-1-150, 150, ISSN 1349-1369, (HAYASHI, Teppei. Non-Constraint Estimation Method of Human Orientation Using Multi-Static MIMO Radar. Proceedings of the 2020 IEICE general conference, communication 1.) page 150, left column | 1-5, 8 |
| Y | page 150, left column | 6-7 |
| Y | JP 2020-153869 A (PANASONIC IP MANAGEMENT CORP.) 24 September 2020 (2020-09-24) paragraphs [0233]-[0240] | 6-7 |
| Y | JP 2020-109390 A (PANASONIC IP MANAGEMENT CORP.) 16 July 2020 (2020-07-16) paragraphs [0050]-[0051] | 7 |
| A | JP 2018-173285 A (KEIO GIJUKU) 08 November 2018 (2018-11-08) entire text, all drawings | 1-8 |
| A | JP 2019-515703 A (ORIGIN WIRELESS, INC.) 13 June 2019 (2019-06-13) entire text, all drawings | 1-8 |

✓ Further documents are listed in the continuation of Box C.  ✓ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **05 August 2022** | **06 September 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT

International application No.

**PCT/JP2022/025557**

### C. DOCUMENTS CONSIDERED TO BE RELEVANT

| Category* | Citation of document, with indication, where appropriate, of the relevant passages | Relevant to claim No. |
|---|---|---|
| A | US 2021/0103031 A1 (INTEL CORP.) 08 April 2021 (2021-04-08)<br>entire text, all drawings | 1-8 |
| A | CN 112859070 A (SICHUAN FIRE RES. INST. MEM) 28 May 2021 (2021-05-28)<br>entire text, all drawings | 1-8 |

Form PCT/ISA/210 (second sheet) (January 2015)

**INTERNATIONAL SEARCH REPORT**
Information on patent family members

International application No.

**PCT/JP2022/025557**

| Patent document cited in search report | | | Publication date (day/month/year) | Patent family member(s) | | | Publication date (day/month/year) |
|---|---|---|---|---|---|---|---|
| JP | 2020-153869 | A | 24 September 2020 | US 2022/0003834 A1 paragraphs [0309]-[0316] DE 112020001356 T5 | | | |
| JP | 2020-109390 | A | 16 July 2020 | US 2020/0213014 A1 paragraphs [0059]-[0060] CN 111381228 A | | | |
| JP | 2018-173285 | A | 08 November 2018 | US 2018/0284223 A1 entire text, all drawings | | | |
| JP | 2019-515703 | A | 13 June 2019 | US 2017/0188359 A1 entire text, all drawings WO 2017/155634 A1 EP 3426148 A1 | | | |
| US | 2021/0103031 | A1 | 08 April 2021 | WO 2019/005936 A1 EP 3646052 A CN 111108408 A | | | |
| CN | 112859070 | A | 28 May 2021 | (Family: none) | | | |

Form PCT/ISA/210 (patent family annex) (January 2015)

**EP 4 365 622 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015042293 A
- JP 2009055997 A
- JP 2007325621 A
- JP 2019093104 A
- JP 2019211458 A
- JP 6504546 B